# EUROPÄISCHE PATENTSCHRIFT

(11) **EP 1 161 922 B1**
(45) Veröffentlichungstag und Bekanntmachung des Hinweises auf die Patenterteilung: **14.12.2005**
(21) Anmeldenummer: 01250202.7
(22) Anmeldetag: 05.06.2001
(51) Int. Cl.: A61B 5/06, A61M 25/01

(54) **System zur Bestimmung der intrakorporalen Lage eines Arbeitskatheters**
System for determination of intracorporal position of an applied catheter
Système pour déterminer la position intracorporelle d'un cathéter

(30) Priorität: 07.06.2000 DE 10027782
(43) Veröffentlichungstag der Anmeldung: 12.12.2001
(73) Patentinhaber: BIOTRONIK Mess- und Therapiegeräte GmbH & Co Ingenieurbüro Berlin, 12359 Berlin (DE)
(72) Erfinder: Ameling, Walter, 52074 Aachen (DE); Adams, Ludwig, 52072 Aachen (DE); Thieling, Lothar, 41812 Erkelenz (DE)
(74) Vertreter: Eisenführ, Speiser & Partner

(56) Entgegenhaltungen:
- WO-A-00/10456
- DE-A- 19 915 689
- GB-A- 2 314 158
- US-A- 5 391 199
- US-A- 5 592 939
- US-A- 5 718 241

## Beschreibung

Die Erfindung betrifft ein System zur Bestimmung der intrakorporalen Lage eines Arbeitskatheters zum Ausführen von gewünschten Arbeitsvorgängen und einem intrakorporalen Referenzkatheter zum Erzeugen eines Koordinatensystems.

Katheter werden in der heutigen Medizin auf vielfache Art und Weise verwendet. Dabei ist es insbesondere von Interesse, die Lage des Katheters auch im implantierten oder eingeschobenen Zustand von außerhalb des Körpers feststellen zu können. Dies gilt insbesondere für Katheter, die zur Behandlung von Herzkrankheiten eingesetzt werden, wobei hier auch eine möglichst genaue Positionsbestimmung des Katheters wichtig ist. Dies gilt sowohl bei dem Sammeln von den Zustand des Herzens beschreibenden Daten, so dass die Daten exakt zugeordnet werden können; dies gilt aber auch für die genaue Bestimmung der Position von Kathetern, die zur Vorbereitung der eigentlichen Therapien in die richtige Position gebracht werden müssen, um die Therapie zu optimieren.

Als ein Beispiel eines Einsatzgebietes des eingangs genannten Systems in der Medizin sei das Vorhofflimmern des Herzens genannt. Diese krankhafte Veränderung ist gekennzeichnet durch eine Vorhoffrequenz von über 350 / Minute und eine völlig unregelmäßige Kammerschlagfolge (absolute Arrhythmie) des Herzens. Das Vorhofflimmern ist, abgesehen von Extrasystolen und Sinustachykardien, die häufigste Rhythmusstörung des Erwachsenenalters mit einer Prävalenz von 0,4 % bei Erwachsenen, die sich auf zwei bis vier Prozent bei über 60jährigen steigert. Das Vorhofflimmern beruht auf mehreren, ständig wechselnden Re-Entry-Erregungskreisen in den Vorhöfen des Herzens. Die Fronten der entsprechenden Erregungswellen kreisen chaotisch um kurz zuvor erregtes und deshalb refraktäres Vorhofmyokard ohne anatomisches Hindernis. Der AV-Knoten wird dabei mit einer Frequenz von 350 bis 600 / Minute aus allen Richtungen mit Erregungsfronten konfrontiert. Die im AV-Knoten stattfindende Leitungsverzögerung führt schließlich dazu, dass die meisten Vorhoferregungen in unterschiedlicher Tiefe im AV-Knoten stecken bleiben und somit nur sehr vereinzelt eine Kammertätigkeit stattfindet. Auf diese Weise kommt es zu einer Verminderung der Herzleistung um bis zu 30 % durch die fehlende Pumpleistung des Vorhofs. Zudem besteht durch die fehlende Pumpleistung die Gefahr der Thrombenbildung im linken Vorhof, die wiederum zu , einem Schlaganfall führen kann.

Diese durch das Vorhofflimmern erhöhte Gefahr des Schlaganfalls sei an dieser Stelle anhand einiger Fallzahlen erläutert. So treten in der Bundesrepublik Deutschland pro Jahr etwa 100.000 neue Schlaganfälle auf. Häufigste Ursache für diese Schlaganfälle sind mit 75 % aller Fälle arterielle Gefäßverschlüsse, die wiederum zu 20 % durch Embolien verursacht werden. Diese Embolien entstehen meistens durch das oben erläuterte Vorhofflimmern, so dass etwa 15 % aller Schlaganfälle, d.h. 15.000 Neuerkrankungen pro Jahr auf Vorhofflimmern zurückzuführen sind. Patienten mit Vorhofflimmern haben somit ein erhöhtes Risiko, einen Schlaganfall zu erleiden. Darüber hinaus ist das Risiko, an einem Schlaganfall zu sterben, bei Patienten mit Vorhofflimmern doppelt so groß wie bei Patienten ohne Vorhofflimmern. Zum Vergleich sei noch erwähnt, dass in den Vereinigten Staaten von Amerika etwa 2 Mio. Menschen mit Vorhofflimmern leben, von denen etwa 600.000 jedes Jahr einen Schlaganfall erleiden, wobei wiederum etwa 160.000 Patienten an diesem Schlaganfall sterben. In den USA ist der Schlaganfall nach Herzkreislaufkrankheiten und Krebs die dritthäufigste Todesursache.

Die bekannten Therapien zur Behandlung des Vorhofflimmerns sind zumeist lediglich palliativ. Das bedeutet, dass zumeist nur die unterschiedlichen Folgen des Vorhofflimmerns behandelt werden, ohne dass die zugrundeliegende Krankheit beseitigt wird. Diese somit nur die Symptome abstellenden Therapien können beispielsweise medikamentös vorgenommen werden, in dem das Vorhofflimmern mit Antikoagulantien behandelt wird. Diese Antikoagulantien vermindern das Risiko der Thrombenbildung und somit das des Schlaganfalls. Derartige Antikoagulantien werden zumeist zusammen mit Antiarrhythmika gegeben. Diese Medikamente können jedoch ihrerseits wiederum das Risiko einer lebensbedrohlichen Kammertachykardie erhöhen. Weiterhin ist es als Therapie bekannt, den AV-Knoten zu durchtrennen und einen Schrittmacher zu implantieren. Diese Maßnahmen dienen der Restaurierung eines normalen Kammerzyklusses. Dabei bleibt jedoch die Ursache, nämlich das Vorhofflimmern und auch das Risiko des Schlaganfalls erhalten.

Als weitere Therapie ist die Implantation eines intrakardialen Defibrillators bekannt. Ein solcher Defibrillator ist in der Lage, das Vorhofflimmern durch Stromschocks, die sogenannte Defibrillation zu terminieren. Auch bei dieser Therapie bleibt jedoch die eigentliche Ursache des Vorhofflimmerns unbehandelt. Zudem wird der meist unvorhergesehene Stromschlag der Defibrillation von den Patienten als sehr unangenehm empfunden.

Als einzige kurative Therapie ist bisher die Maze-Prozedur bekannt, bei der in einem chirurgischen Eingriff am offenen Herzen elektrisch isolierendes, lineares Narbengewebe im Endokard erzeugt wird. Dieses Narbengewebe verhindert das Entstehen von Kreiserregungen und somit das Entstehen von Vorhofflimmern. Es wird somit die Pumpfunktion des Vorhofes erhalten und der Kammerrhythmus des Herzens normalisiert. Diese Operation ist jedoch sehr aufwendig und mit einer hohen Mortalität und Morbidität verbunden.

Um diese Schwierigkeiten zu umgehen, wurden im Stand der Technik die eingangs genannten Systeme entwickelt. Dabei wird mit Hilfe von Ablationskathetern die zuvor beschriebene Therapie minimal invasiv durchgeführt. Ein derartiges System ist beispielsweise aus der US 5,718,241 bekannt. Gegenstand dieses Patents ist die Lagevermessung arrhythmogener Zonen mittels Ablationskatheter und die Positionsbestimmung des Katheters über Referenzfilter. Zur Erzeugung eines elektrischen Abbilds aufgrund von Refraktärzeit- und Reizleitungsgeschwindigkeitsmessungen soll die Position eines Ablationskatheters mit einer Tip-Elektrode beispielsweise anhand eines Referenzfeldes bestimmt werden. Als Ergebnis dieses Mappings soll ein geometrisches Maß ("dimension value") für Zonen mit bestimmten Reizleitungseigenschaften bestimmt werden, anhand dessen Größe und Position der zur erzeugenden Läsionen bestimmt wird. Da diese bekannte Positionsvermessung einer Tip-Elektrode nur sukzessive, d.h. Punkt für Punkt durchgeführt werden kann, kann die Geometrie und die dazu gehörige elektrische Aktivität der entsprechenden Herzkavität auch nur Punkt für Punkt bestimmt werden. Darüber hinaus ist Voraussetzung, dass eine stationäre, vom Patienten gut tolerierte Arrhythmie vorliegt, da prinzipbedingt nicht zeitgleich an verschiedenen Stellen des Endokards gemessen werden kann und die Prozedur somit zeitaufwendig ist. Auch die Erzeugung der linearen Läsion ist wiederum zeitaufwendig, da diese nur Punkt für Punkt erzeugt werden kann.

Aufgabe der vorliegenden Erfindung ist es daher, die Einsetzbarkeit des eingangs genannten Systems zu verbessern.

Diese Aufgabe wird bei einem System der eingangs genannten Art dadurch gelöst, dass der Arbeitskatheter mehrere Referenzeinheiten aufweist, die von der Sensorvorrichtung erfassbar sind.

Die Erfindung weist gleich eine ganze Reihe von Vorteilen auf. Zunächst ist es dank der Erfindung möglich, eine genaue Bestimmung der Position und auch der Orientierung und des räumlichen Verlaufs des Arbeitskatheters vorzunehmen. Bei dem Arbeitskatheter kann es sich sowohl um einen Mappingkatheter handeln, der mit Hilfe von auf ihm angebrachten Elektroden die elektrische Aktivität der entsprechenden Herzregion vermisst; es kann sich jedoch auch um einen Ablationskatheter handeln, der die entsprechenden Regionen mit einer Läsion versieht. In beiden Fällen ist jedoch dank der Erfindung eine exakte Positionierung, Orientierung und Festlegung des räumlichen Verlaufs des entsprechenden Katheters nicht nur bezüglich seiner Spitze, sondern bezüglich des gesamten distalen Abschnittes möglich, auf welchem sich Referenzeinheiten befinden, die von der Sensorvorrichtung des Referenzkatheters erfassbar sind. Es ist somit eine deutliche Erhöhung der Ortsauflösung möglich. Dies ermöglicht wiederum ein hochauflösendes Mapping aller Herzkavitäten, in denen sich der Arbeitskatheter befindet. Weiterhin ist es vorteilhaft, dass die präzise Positionierung des Arbeitskatheters auf bewährten Kathetern aufbauen kann, so dass die von bewährten Kathetern bekannte Patientensicherheit, Positioniersicherheit, Handhabung und Zulassung weiter besteht. Die zielgenaue Positionierung des Ablationskatheters nach einem zielgenauen und hochauflösendem Mapping des interessierenden Endokard stellt somit eine hervorragende Alternative zu der eingangs genannten erwähnten Maze-Operation dar, wobei gleichzeitig deutlich reduzierte Mortalitäts- und Morbiditätsraten erreicht werden können. Darüber hinaus sind gegenüber der Maze-Operation dank der Erfindung trotz deutlich höherer Genauigkeit des Eingriffs mit einem derartigen Eingriff erheblich niedrigere Kosten verbunden.

Das erfindungsgemäße System ermöglicht dem Arzt somit eine genaue Beurteilung der Erregungsmechanismen des zu behandelnden Endokard und ermöglicht ihm eine genaue Planung der Therapie, nämlich die genaue Festlegung der Verläufe der mit Hilfe des Ablationskatheters aufzubringenden Läsionslinien. Auch die Übertragung des schließlich vom Arzt auszuarbeitenden Therapieplans auf den Patienten kann mit Hilfe des erfindungsgemäßen Systems erheblich verbessert werden, da der Ablationskatheter zum Aufbringen der Läsionslinien ebenso präzise vermessen werden kann wie die Anatomie, so dass eine zielgenaue Positionierung des Ablationskatheters und somit ein zielgenaues Aufbringen der Läsionslinien möglich ist.

Darüber hinaus ist es dank der Erfindung möglich, auch bei kurzzeitig auftretenden oder vom Patienten schlecht tolerierten Tachykardien gleichzeitig an verschiedenen Orten mit Hilfe des Arbeitskatheters und seiner Referenzeinheiten Signale aufzunehmen, d.h. das Mapping durchzuführen. Es entsteht somit bereits mit einem einzigen Katheter, der alternativ auch als Korbkatheter ausgebildet sein kann, ein dreidimensionales Mapping der interessierenden Herzregion, ohne dass dies einen großen, bei einer solchen Tachykardie nicht tolerierbaren Zeitaufwand erfordert.

Es lässt sich somit festhalten, dass das erfindungsgemäße System ein genaues, dreidimensionales Abbild der zu untersuchenden Herzkavität und eine darauf bezogene Darstellung der elektrischen Aktivität durch das zur Verfügungstellen mehrerer Referenzeinheiten auf dem Arbeitskatheter ermöglicht. Darüber hinaus verfügt man dank des erfindungsgemäßen Systems über einen Ablationskatheter, der dank des erfindungsgemäßen Systems ohne Neupositionierung lineare Läsionen erzeugen kann, da er mit Hilfe des Systems präzise und ohne zusätzliche röntgenologische Maßnahmen platziert werden kann, in dem der von dem Referenzkatheter erfasste Verlauf des Ablationskatheters beispielsweise kontinuierlich in eine dreidimensionale Darstellung der entsprechenden Herzkavität, welche Darstellung beispielsweise durch einen entsprechenden Mappingkatheter zur Verfügung gestellt werden kann, eingeblendet wird.

Eine weitere Ausführungsform der Erfindung zeichnet sich dadurch aus, dass es sich bei dem Arbeitskatheter um einen fest in einen Körper implantierbaren, Elektroden eines Herzschrittmachers oder eines Defibrillators tragenden Katheter handelt. Die Vorteile dieser Ausführungsform liegen insbesondere darin, dass mit Hilfe des intrakorporalen Referenzkatheters des Systems eine exakte Bestimmung der intrakorporalen Lage des die Elektroden tragenden Katheters möglich ist, ohne dass es zu einer Strahlenbelastung durch Röntgen des Patienten kommt.

Eine weitere bevorzugte Ausführungsform der Erfindung zeichnet sich dadurch aus, dass die Referenzeinheiten auf dem Arbeitskatheter derart angeordnet sind, dass die Position und/oder die Orientierung des Arbeitskatheters in dem Koordinatensystem von der Sensorvorrichtung erfassbar ist. Mit Hilfe dieser Ausführungsform ist es somit nicht nur möglich, die genaue Stellung im Körper festzustellen, sondern auch eine mögliche Rotation des Katheters, und somit eine mögliche Änderung der Orientierung oder des räumlichen Verlaufs auf ihm angebrachter Ablationsmittel oder anderer räumlich zu positionierender Arbeitsmittel festzustellen. Auf diese Weise wird daher eine in drei Dimensionen präzise Ausrichtung des derartig mit Referenzeinheiten ausgestatteten Katheters erreicht.

Eine weitere bevorzugte Ausführungsform der Erfindung zeichnet sich dadurch aus, dass es sich bei den Referenzeinheiten um auf oder in dem Katheter angebrachte Ultraschallkristalle oder Spulen handelt. Auf diese Weise lassen sich die Referenzeinheiten besonders einfach realisieren.

Eine weitere bevorzugte Ausführungsform der Erfindung zeichnet sich dadurch aus, dass mindestens eine Referenzeinheit an der Katheterspitze angeordnet ist, während der mindestens eine weitere Referenzeinheit im distalen Bereich des Katheters angeordnet ist, wobei im distalen Bereich bevorzugt eine ganze Reihe von Referenzeinheiten angeordnet ist, weiter bevorzugt 12 bis 24 Referenzeinheiten. Die Vorteile dieser Ausführungsformen mit mehreren Referenzeinheiten liegen darin, dass mit der erhöhten Anzahl von Referenzeinheiten besonders in dem wichtigen distalen Bereich des Katheters eine erhöhte Genauigkeit des Mappings - oder wenn es sich bei dem Kathetern um einen Ablationskatheter handelt - des Aufbringens der Läsion gesichert ist.

Eine weitere bevorzugte Ausführungsform der Erfindung zeichnet sich dadurch aus, dass der distale Bereich des Arbeitskatheters eine zuvor festgelegte, bestimmte Form, bevorzugt die eines Kreisbogens, aufweist, auf welchem distalen Bereich mindestens drei Referenzeinheiten verteilt sind, so dass von der Sensorvorrichtung bei der Ermittlung der Lage des Arbeitskatheters bei der Positionsberechnung des Arbeitskatheters die bestimmte, zuvor festgelegte Form des distalen Bereiches einbezogen werden kann. Die Vorteile dieser Ausführungsform liegen insbesondere darin, dass bereits mit einer geringen Anzahl von Referenzeinheiten, beispielsweise drei Referenzeinheiten, und der Verwendung eines eine bestimmte Form aufweisenden distalen Bereiches, beispielsweise eines distalen Bereiches, der die Form eines Kreisbogens aufweist, eine genaue Bestimmung der Position und Orientierung des entsprechenden Arbeitskatheters möglich ist.

Eine weitere bevorzugte Ausführungsform der Erfindung zeichnet sich dadurch aus, dass es sich bei dem Referenzkatheter ebenfalls um einen Arbeitskatheter handeln kann, oder es sich bei dem Arbeitskatheter ebenfalls um einen Referenzkatheter handeln kann, in dem auf jeden Katheter jeweils Referenzeinheiten zum Senden und Referenzeinheiten zum Empfangen von Wellen vorgesehen und/oder jeweils Referenzeinheiten vorgesehen sind, die Wellen gleichzeitig senden und empfangen können. Die Vorteile dieser Ausführungsform liegen insbesondere darin, dass ein mit derartig flexibel einsetzbaren Kathetern ausgestattetes System ebenfalls flexibler einsetzbar ist. So kann bei dieser Ausführungsform bei Bedarf die Position des Referenzkatheters bestimmt werden, in dem der Arbeitskatheter als Referenzkatheter arbeitet und umgekehrt.

Eine weitere bevorzugte Ausführungsform der Erfindung zeichnet sich dadurch aus, dass die Sensorvorrichtung mit Hilfe der erfindungsgemäßen Referenzeinheiten eine topologische und/oder eine elektrische Vermessung des Endokard vornehmen kann, in dem sich der jeweilige Arbeitskatheter befindet. Die Vorteile dieser Ausführungsform liegen insbesondere darin, dass somit nacheinander oder gleichzeitig die Präzision der mit mehreren Referenzeinheiten versehenen erfindungsgemäßen Arbeitskatheter genutzt werden kann, um eine präzise Bestimmung der Anatomie des Endokard und eine präzise elektrische Vermessung der entsprechenden Herzkavität vornehmen zu können.

Eine weitere bevorzugte Ausführungsform der Erfindung zeichnet sich dadurch aus, dass die Sensorvorrichtung die Lage des Arbeitskatheters in dem von dem Referenzkatheter aufgespannten Koordinatensystem mittels eines elektrischen Verarbeitungsmittels ermittelt, in dem die Sensorvorrichtung mit Hilfe des Referenzkatheters mindestens ein elektromagnetisches und/oder ein Ultraschall-Feld aufbaut. Mit Hilfe eines elektromagnetischen Feldes lässt sich das erfindungsgemäße System besonders einfach realisieren.

Eine weitere bevorzugte Ausführungsform der Erfindung zeichnet sich dadurch aus, dass der Referenzkatheter bei Einsatz des Systems im Herzen bevorzugt im Koronarsinus platzierbar ist. Bei dieser Ausführungsform ist es besonders vorteilhaft gewährleistet, dass der Referenzkatheter des erfindungsgemäßen Systems automatisch die Bewegungen des Patienten und des Patientenherzens kompensiert. Die Ausführung der Arbeitsvorgänge mit Hilfe des Arbeitskatheters, beispielsweise das Ausführen eines Mappingvorganges oder eines Ablationsvorganges geschieht somit bezüglich eines im Herzen selbst liegenden, also mitbewegten Referenzsystems. Ein zusätzlicher Katheter, der die Bewegung des Herzens oder des Patienten erfasst, ist somit nicht mehr notwendig.

Eine weitere bevorzugte Ausführungsform der Erfindung zeichnet sich dadurch aus, dass die Sensorvorrichtung derart ausgebildet ist, dass sie aus den mindestens drei Referenzeinheiten des Arbeitskatheters einen dreidimensionalen Spline berechnet, der die Lage des Arbeitskatheters in dem von dem Referenzkatheter aufgespannten Koordinatensystem darstellt. Die Vorteile dieser Ausführungsform liegen darin, dass der derart berechnete Spline einem zuvor mit Hilfe eines Arbeitskatheters erfassten Bild der Anatomie des Endokard überlagert werden kann, so dass in einem entsprechenden Anzeigesystem, beispielsweise auf dem Monitor, der das System bedienenden Person die genaue Lage des Arbeitskatheters in der Herzkavität dargestellt werden kann.

Eine weitere bevorzugte Ausführungsform der Erfindung zeichnet sich dadurch aus, dass die Steuervorrichtung und/oder die Sensorvorrichtung in den jeweiligen Kathetern vorgesehen sind. Der Vorteil dieser Ausführungsform liegt darin, dass auf diese Weise bereits die jeweiligen Katheter selber voll integriert alle notwendigen Arbeitsmittel enthalten.

Eine weitere bevorzugte Ausführungsform der Erfindung zeichnet sich dadurch aus, dass mindestens einer der Referenzeinheiten als Sensor zur Erfassung des Vorhandenseins und/oder der Stärke des Wandkontaktes einer Elektrode des Arbeitskatheters mit dem den Katheter umgebenden Endokard ausgebildet ist. Die Vorteile dieser Ausführungsform liegen darin, dass durch die Feststellung des endokardialen Wandkontaktes der einzelnen Elektrode eine Aussage darüber getroffen werden kann, ob die entsprechende Elektrode des Arbeitskatheters am Endokard anliegt oder nicht, so dass beurteilt werden kann, ob die entsprechenden Referenzeinheiten zur genauen Erfassung der Anatomie es Endokards benutzt werden können. Liegen die Elektroden des Arbeitskatheters, die gleichzeitig als Referenzeinheiten dienen können, am Endokard an, geschieht die Erfassung der Anatomie des Endokards dadurch, dass in dem Bereich des Katheters, der den Wandkontakt mit dem Endokard hat, die durch die mit dem Endokard in Kontakt stehenden Referenzeinheiten gebildete Raumkurve zur Generierung einer dreidimensionalen Oberfläche des Endokard verwendet wird.

Eine weitere bevorzugte Ausführungsform der Erfindung zeichnet sich dadurch aus, dass das System mindestens zwei, bevorzugt fünf, Arbeitskatheter aufweist, wobei jeder Katheter mindestens drei, bevorzugt 12 bis 24 Referenzeinheiten, die weiter bevorzugt als Elektroden, noch weiter bevorzugt als Ringelektroden ausgebildet sind, aufweist, um so die entsprechende Anzahl von Potentialverläufen bei in einer Herzkavität eingeschobenen Arbeitskathetem zu erfassen. Die Vorteile dieser Ausführungsform der Erfindung liegen insbesondere darin, dass auf diese Weise die Anatomie und die zugehörige Erregung der entsprechenden Herzregion, erfasst werden können. Auch können auf diese Weise Untersuchungen der Herzdynamik und transienter Vorgänge der Erregung realisiert werden. Das Zusammenfügen mehrerer Katheter mit mehreren bevorzugt als Polringe ausgebildeten, und als Referenzeinheiten dienenden Elektroden längs einer Raumkurve bildet quasi einen virtuellen Korbkatheter, so dass trotz Verwendung einfacher linearer Katheter die Vorteile eines Korbkatheters, insbesondere die Möglichkeit einer Momentaufnahme der Herzerregung, wie sie beispielsweise besonders bei kurzzeitig auftretenden Tachykardien notwendig ist, ermöglicht werden.

Eine weitere bevorzugte Ausführungsform der Erfindung zeichnet sich dadurch aus, dass der Arbeitskatheter mit einer Anzahl von mindestens zwei bevorzugt als Ringelektroden ausgebildeten Elektroden versehen ist, die örtlich verschieden von den Referenzeinheiten auf dem Arbeitskatheter angebracht sind, wobei die Referenzeinheiten zu den Elektroden eine zuvor festgelegte, bestimmte räumliche Lage aufweisen, die von der Sensorvorrichtung bei der Ermittlung der Lage des Arbeitskatheters in dem von dem Referenzkatheter aufgespannten Koordinatensystem berücksichtigbar ist. Die Vorteile dieser Ausführungsform liegen insbesondere darin, dass durch die Trennung von Referenzeinheiten und Elektroden mögliche Wechselwirkungen zwischen beiden elektromagnetisch arbeitenden Bestandteilen der Arbeitskatheter ausgeschlossen werden können.

Eine weitere bevorzugte Ausführungsform der Erfindung ist ein System, welches einen als Anzeigevorrichtung dienenden Monitor zur Anzeige der von der Sensorvorrichtung ermittelten Lage des Arbeitskatheters in dem von dem Referenzkatheter erzeugten Koordinatensystem enthält. Von einem solchen Monitor kann sowohl die von einem Mappingkatheter ermittelte dreidimensionale Struktur der Endokardoberfläche als Abwicklung oder als dreidimensionales Objekt dargestellt werden. Eine solche Abwicklung oder ein solches dreidimensionales Objekt kann dann vom Benutzer manipuliert, zum Beispiel gedreht werden, damit der Benutzer alle Seiten betrachten kann. Eine solche 3-D-Struktur stellt somit die Anatomie des entsprechenden Endokards dar. Auf eine derartige Endokardstruktur kann aber auch - wie oben bereits erwähnt - die Darstellung der von einem Mappingkatheter gemessenen Erregung der entsprechenden Herzkavität oder der bereits eingeführte dreidimensionale Spline des erfassten Ablationskatheters projiziert werden. Die Erregung kann beispielsweise in Form eines aus dem Mappingkatheter gemessenen Potentialen berechneten Isochronenbildes dargestellt werden. Ein solches Isochronenbild stellt die Aktivierungszeit auf der Endokardoberfläche dar. Mit Hilfe von Triggeralgorhitmen können dann aus den gemessenen Potentialläufen der Beginn der Erregung an jeder Elektrode des Mappingkatheters berechnet werden. Diese an einzelnen Stellen des Endokards berechneten Zeiten werden dann für das restliche Endokard interpoliert und dargestellt, so dass jeder Aktivierungszeit beispielsweise eine Farbe entspricht. Man kann somit für jeden Herzschlag des Herzens ein Isochronenbild erhalten.

Bei einem anderen Verfahren, der sogenannten Potentialdarstellung, wird das gemessene Potential direkt farblich dargestellt. Jedem Potentialwert wird hier ebenfalls eine Farbe zugeordnet, so dass durch geeignete Farbwahl gut unterschieden werden kann zwischen den verschiedenen Zuständen, beispielsweise "nicht erregt", "Erregung", "beginnt", und "erregt". Dabei werden zwischen den Elektroden die Werte bei der Isochronendarstellung interpoliert. Dabei hat es sich als sinnvoll erwiesen, die Darstellung der Potentiale in einer Animationssequenz in Zeitlupe vorzunehmen, da die Erregungswelle in Echtzeit sehr schnell über das Endokard läuft, so dass eine zeitsynchrone Darstellung wenig sinnvoll ist, da nur schwer von dem Benutzer nachvollziehbar ist.

Weitere bevorzugte Ausführungsformen der Erfindung sind in den Unteransprüchen angegeben.

Die Erfindung soll nun anhand von Ausführungsbeispielen mit Hilfe der Figuren näher erläutert werden. Diese zeigen:
- Fig. 1: ein System zur Bestimmung der intrakorporalen Lage eines Arbeitskatheters gemäß einem ersten Ausführungsbeispiel;
- Fig. 1a: einen Querschnitt durch den Arbeitskatheter von Figur 1;
- Fig. 2: eine detaillierte Darstellung eines Längsschlitz des distalen Endes eines Arbeitskatheters gemäß einem zweiten Ausführungsbeispiel; und
- Fig. 2a: einen Querschnitt durch den Arbeitskatheter von Fig. 2.

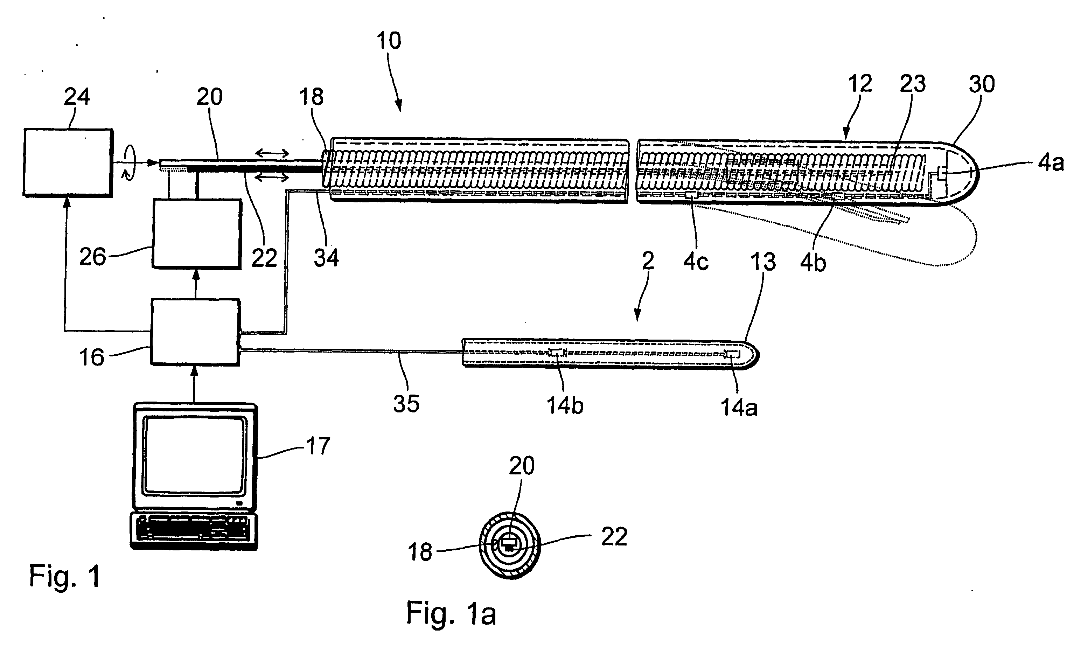

In Fig. 1 ist ein System zur Bestimmung der intrakorporalen Lage eines Arbeitskatheters 10 gezeigt. Das System weist dabei einen Arbeitskatheter 10 und ein Referenzkatheter 2 auf. Sowohl der Arbeitskatheter 10 als auch der Referenzkatheter 2 sind intrakorporal einführbar ausgestaltet. Der Arbeitskatheter 10 weist dabei drei Referenzeinheiten 4a, 4b und 4c auf während der Referenzkatheter 2 zwei Referenzeinheiten 14a und 14b aufweist. Die Referenzeinheiten 4a - 4c und 14a, 14b sind dazu ausgestaltet, Ultraschall-Wellen oder elektromagnetische Wellen zu empfangen und/oder zu senden.

Figur 1 zeigt insbesondere einen Arbeitskatheter 10 gemäß einem ersten Ausführungsbeispiel, dessen distales Ende 12 in beliebiger radialer Richtung durch Deflektion seitlich auslenkbar ist. Diese Auslenkung erfolgt beispielsweise nach dem aus der US 5,254,088 bekannten Prinzip. Der Arbeitskatheter 10 weist dazu an seinem proximalen Ende zwei mechanische Stellantriebe bzw. Aktuatoren 24 und 26 auf, die mit einer ein Lumen einschließenden und an ihrem distalen Ende flexiblen Spiralhülle 18 sowie zwei in dem Lumen der Spiralhülle 18 geführten Steuerdrähten 20 und 22 verbunden sind. Die beiden mechanischen Stellantriebe bzw. Aktuatoren 24 und 26 sind in bekannter Weise derart mit den beiden Steuerdrähten 20 und 22 verbunden, dass eine Rotation der Führungsdrähte 20 und 22 bezüglich des übrigen Arbeitskatheters 10 und eine axiale Bewegung der Steuerdrähte 20 und 22 relativ zueinander ermöglich wird, wobei die beiden Steuerdrähte 20 und 22 an ihrem distalen Ende 23 miteinander verbunden sind. Durch das axiale Verschieben der Steuerdrähte relativ zueinander mittels des Aktuators 26 kommt es zu einer seitlichen Ausbiegung der Spiralhülle 18 und damit des Arbeitskatheters 10 im flexiblen Bereich der Spiralhülle 18 am distalen Ende 12 des Arbeitskatheters 10. Eine Rotation der Führungsdrähte 20 und 22 ist mittels des Aktuators 24 möglich.

Die Spiralhülle 18 ist ferner in dem Arbeitskatheter 10 relativ zum Arbeitskatheter 10 rotierbar angeordnet. Durch eine Rotationsbewegung der Spiralhülle 18 mit den darin geführten Steuerdrähten 20 und 22 bezüglich des Arbeitskatheters 10 kann die radiale Richtung der seitlichen Auslenkung beim Deflektieren des distalen Katheterendes 12 bestimmt werden.

Der Arbeitskatheter 10 weist drei Referenzeinheiten 4a, 4b und 4c auf. Dabei befindet sich die erste Referenzeinheit 4a an der distalen Spitze 30 des Katheters, während sich der zweite und die dritte Referenzeinheit 4b und 4c im distalen Bereich 12 des Katheters befindet. Diese Referenzeinheiten können beispielsweise als Wandlereinheiten insbesondere als Ultraschall-Wandlereinheiten, Ultraschallkristalle bzw. Piezo-Kristalle oder Spulen ausgestaltet und dazu geeignet sein, Ultraschall-Wellen oder elektromagnetische Wellen zu erzeugen oder zu empfangen. Die drei Referenzeinheiten 4a, 4b und 4c sind über eine Signalleitung 34 mit einer Steuereinheit 16 verbunden. Natürlich sind auch mehr als zwei Referenzeinheiten 4b und 4c im distalen Bereich 12 des Arbeitskatheters 10 möglich. Beispielsweise können 12 bis 24 Referenzeinheiten im distalen Bereich 12 des Arbeitskatheters 10 vorgesehen sein.

Neben dem Arbeitskatheter 10 ist in Figur 1 auch ein Referenzkatheter 2 gezeigt. Dieser Referenzkatheter 2 weist dabei zwei Referenzeinheiten 14a und 14b in seinem distalen Bereich 13 auf. Dabei ist eine Referenzeinheit 14a an der distalen Spitze angeordnet, während eine zweite Referenzeinheit 14b im distalen Bereich 13 des Referenzkatheters 2 angeordnet ist. Diese beiden Referenzeinheiten 14a und 14b sind über eine weitere Signalleitung 35 mit der Steuereinheit 16 verbunden. Die beiden Referenzeinheiten 14a und 14b sind ebenfalls wie die Referenzeinheiten 4a, 4b und 4c dazu geeignet Ultraschall-Wellen oder elektromagnetische Wellen zu erzeugen und/oder zu empfangen.

Die Referenzeinheiten 4a - 4c sowie 14a und 14b sind an vorab festgelegten Stellen in dem Arbeitskatheter 10 bzw. dem Referenzkatheter 2 angeordnet, so dass die relative Position der jeweiligen Referenzeinheiten 4a - 4c sowie der jeweiligen Referenzeinheiten 14a und 14b zueinander bekannt ist und bei der Positionsberechnung mit berücksichtigt werden können.

Die Steuereinheit 16 empfängt Signale von den Referenzeinheiten des Arbeitskatheters 10 und des Referenzkatheters 2, wobei entweder die Referenzeinheiten 14a, 14b des Referenzkatheters 2 Ultraschall-Wellen oder elektromagnetische Wellen aussenden und die Referenzeinheiten 4a - 4c des Arbeitskatheters 10 diese Wellen empfangen oder die Referenzeinheiten 4a - 4c des Arbeitskatheters 10 die Wellen aussenden und die Referenzeinheiten 14a, 14b des Referenzkatheters 2 die Wellen empfangen. Mit Hilfe dieser empfangenen Signale berechnet die Steuereinheit 16 die relative Position des Arbeitskatheters 10 bezüglich des Referenzkatheters 2. Anhand dieser ermittelten Positionsdaten berechnet die Steuereinheit 16 ferner ein Steuersignal für die Aktuatoren 24 und 26 zum Steuern der Bewegung des Arbeitskatheters 10 . Mit Hilfe der Steuereinheit 16 lässt sich somit ein geschlossenes bzw. rückgekoppeltes Steuersystem ausbilden, durch das der Arbeitskatheter 10 automatisch zu einer gewünschten Position bewegt werden kann. Dabei ist es insbesondere vorteilhaft, wenn die Steuereinheit 16 programmierbar ausgestaltet ist, so dass eine gewünschte intrakorporale Position eingegeben werden kann, zu der der Arbeitskatheter 10 kontrolliert mit Hilfe des Referenzkatheters 2 bewegt wird. Insbesondere kann die Steuereinheit 16 aus den drei Referenzeinheiten 4a - 4c des Arbeitskatheters 10 einen dreidimensionalen Spline berechnen, der die Lage des Katheters relativ zu dem Referenzkatheter darstellt.

In einer nicht dargestellten, vereinfachten Ausführungsvariante ist die Steuereinheit ausgebildet, die Lage des Arbeitskatheters relativ zum Referenzkatheter anzuzeigen und der Arbeitskatheter von Hand steuerbar ausgeführt, so dass der Arzt den Arbeitskatheter anhand der Anzeige von Hand steuerbar steuern kann.

Das erfindungsgemäße System weist ferner einen Computer nebst Monitor 17 auf, auf dem die eine mittels der Steuereinheit 16 ermittelte dreidimensionale Struktur der Endokardoberfläche bzw. die relative Position des Arbeitskatheters 10 angezeigt werden kann.

Figur 1a zeigt einen Querschnitt des in Figur 1 gezeigten Arbeitskatheters 10. Gezeigt sind hierbei die Spiralhülle 18 und die beiden Steuerdrähte 20 und 22. Dabei ist der Steuerdraht 20 als ein Flachband ausgestaltet, wodurch die Rotation des Katheters vereinfacht wird.

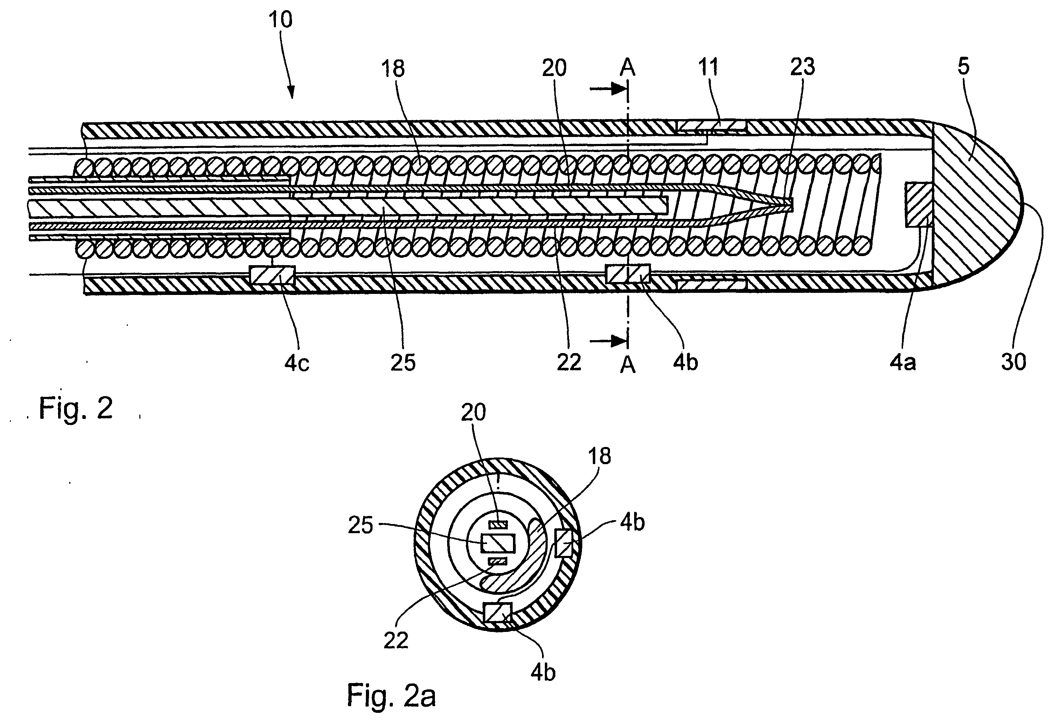

In Figur 2 ist der distale Bereich 12 eines Arbeitskatheters 10 gemäß einem zweiten Ausführungsbeispiel gezeigt. Dabei entspricht der Aufbau des distalen Endes 12 des Arbeitskatheters 10 gemäß dem zweiten Ausführungsbeispiel dem Aufbau des distalen Endes 12 des Arbeitskatheters 10 gemäß dem ersten Ausführungsbeispiel in Figur 1. An der distalen Spitze 30 des Arbeitskatheters 10 ist dabei eine Tip-Elektrode 5 vorgesehen. Der Katheter weist ferner eine Ringelektrode 11 in seinem distalen Bereich 12 auf. Die Tip-Elektrode 5 und die Ringelektrode 11 können hierbei Elektroden zum Mappen und/oder zur Ablation von Gewebe darstellen. Vorzugsweise können auch weitere Elektroden, insbesondere Ringelektroden, im distalen Bereich 12 des Arbeitskatheters 10 angeordnet werden. Diese Elektroden können einzelnen, gemeinsam oder in verschiedenen Kombinationen zum Mappen und/oder zur Ablation von Gewebe angesteuert werden.

Fig. 2a zeigt einen Querschnitt entlang des Schnittes AA im distalen Bereich 12 des Arbeitskatheters 10 aus Figur 2. Der Aufbau des Querschnitts entspricht dabei im wesentlichen dem Aufbau des Querschnitt aus Figur 1a. Zwischen den Steuerdrähten 20 und 22 ist jedoch ein Flachband 25 angeordnet. Die drei Referenzeinheiten 4a, 4b des Katheters bilden im wesentlichen ein Dreieck, so dass eine exakte dreidimensionale Positionsbestimmung möglich wird.

## Patentansprüche

1. System zur Bestimmung der intrakorporalen Lage eines Arbeitskatheters (10), mit
einem Arbeitskatheter (10), der dazu ausgestaltet ist, gewünschte Arbeitsvorgänge auszuführen, und
einem intrakorporalen Referenzkatheter (2), der dazu ausgestaltet ist, ein Koordinatensystem zu erzeugen,
**dadurch gekennzeichnet, dass** der Arbeitskatheter (10) mehrere Arbeitskatheter-Referenzeinheiten (4a-c) aufweist, die dazu ausgestaltet sind, für die Position des Arbeitskatheters (10) charakteristische Signale zu senden, und
der Referenzkatheter (3) mehrere Referenzkatheter-Referenzeinheiten (14) aufweist, die dazu ausgestaltet sind, die von den Arbeitskatheter-Referenzeinheiten (4) gesendeten Signale zu empfangen,
das System ferner eine Verarbeitungseinheit (16) aufweist, die dazu ausgestaltet ist, die Position und die intrakorporale Ausrichtung des Arbeitskatheters (10) anhand von den Referenzkatheter-Referenzeinheiten (14a,b) empfangenen Signalen zu berechnen.

2. System nach Anspruch 1,
wobei der Arbeitskatheter (10) ein Mappingkatheter zum Erstellen eines dreidimensionalen Abbildes der den Mappingkatheter umgebenden Herzkavität ist.

3. System nach einem der vorstehenden Ansprüche,
wobei der Arbeitskatheter (10) ein Ablationskatheter zum Erzeugen einer, bevorzugt linearen, Läsion des den Ablationskatheter umgebenden Endokards ist.

4. System nach einem der vorstehenden Ansprüche,
wobei es sich bei dem Arbeitskatheter (10) um einen fest in einen Körper implantierbaren, Elektroden eines Herzschrittmachers oder eines Defibrillators tragenden Katheter handelt.

5. System nach einem der vorstehenden Ansprüche,
wobei die Arbeitskatheter-Referenzeinheiten (4a-c) auf dem Arbeitskatheter (10) derart unsymmetrisch, bevorzugt die Ecken eines Dreiecks bildend, angeordnet sind, dass die Orientierung des Arbeitskatheters (10) in dem Koordinatensystem des Referenzkatheters (2) erfassbar ist.

6. System nach einem der vorstehenden Ansprüche,
wobei es sich bei den Referenzeinheiten (4, 14) um auf oder in dem Katheter angebrachte Ultraschallkristalle oder Spulen handelt.

7. System nach einem der vorstehenden Ansprüche,
wobei mindestens eine Referenzeinheit (4a) an der Katheterspitze (30) angeordnet ist, während mindestens eine weitere Referenzeinheit (4b,c) im übrigen distalen Bereich (12) des Katheters (10) angeordnet ist, wobei im distalen Bereich (12) bevorzugt eine ganze Reihe von Referenzeinheiten angeordnet ist, weiter bevorzugt 12 bis 24 Referenzeinheiten.

8. System nach einem der vorstehenden Ansprüche,
wobei der distale Bereich (12) des Arbeitskatheters (10) eine zuvor festgelegte, bestimmte Form, bevorzugt die eines Kreisbogens, aufweist, auf welchem distalen Bereich mindestens drei Referenzeinheiten (4) verteilt sind, so dass von der Verarbeitungseinheit (16) bei der Ermittlung der Lage des Arbeitskatheters (10) bei der Positionsberechnung des Arbeitskatheters (10) die bestimmte, zuvor festgelegte Form des distalen Bereiches (12) einbeziehbar ist.

9. System nach einem der vorstehenden Ansprüche,
wobei es sich bei dem Referenzkatheter (2) ebenfalls um einen Arbeitskatheter (10) handelt, oder es sich bei dem Arbeitskatheter (10) ebenfalls um einen Referenzkatheter (2) handelt, indem auf jeden Katheter jeweils Referenzeinheiten (4, 14) zum Senden und Referenzeinheiten (4) zum Empfangen von Wellen, bevorzugt elektromagnetischer und/oder Ultraschall-Wellen, vorgesehen und/oder jeweils Referenzeinheiten (4, 14) vorgesehen sind, die Wellen, bevorzugt elektromagnetische Wellen, gleichzeitig senden und empfangen können.

10. System nach einem der vorstehenden Ansprüche,
wobei die Verarbeitungseinheit (16) dazu ausgestaltet ist, mit Hilfe der Referenzeinheiten (4) eine topologische und/oder eine elektrische Vermessung des Endokard vorzunehmen, in dem sich der jeweilige Arbeitskatheter (10) befindet.

11. System nach einem der vorstehenden Ansprüche,
wobei die Referenzkatheter-Referenzeinheiten (14a,b) dazu ausgebildet ist, elektromagnetische Strahlung und/oder Ultraschall-Wellen abzustrahlen, um die Lage des Arbeitskatheters (10) in dem von dem Referenzkatheter (2) aufgespannten Koordinatensystem zu ermitteln, wobei die Referenzkatheter-Referenzeinheiten (14a,b) mindestens ein elektromagnetisches Feld aufbauen.

12. System nach einem der vorstehenden Ansprüche,
wobei der Referenzkatheter (2) bei Einsatz des Systems im Herzen bevorzugt im Koronarsinus platziert ist.

13. System nach einem der vorstehenden Ansprüche,
wobei die Verarbeitungseinheit (16) ausgestaltet ist, aus den Daten von den mindestens drei Arbeitskatheter-Referenzeinheiten (4a-c) einen dreidimensionalen Spline zu berechnen, der die Lage des Arbeitskatheters (10) in dem von dem Referenzkatheter aufgespannten Koordinatensystem darstellt.

14. System nach einem der vorstehenden Ansprüche,
wobei die Verarbeitungseinheit (16) in den jeweiligen Kathetern integriert sind.

15. System nach einem der vorstehenden Ansprüche,
wobei mindestens einer der Referenzeinheiten als Sensor zur Erfassung des Vorhandenseins und/oder der Stärke des Wandkontaktes des Arbeitskatheters (10) mit dem den Katheter umgebenden Endokard ausgebildet ist.

16. System nach einem der vorstehenden Ansprüche,
wobei das System mindestens zwei, bevorzugt fünf, Arbeitskatheter (10) aufweist, wobei jeder Katheter mindestens drei, bevorzugt 12 bis 24 Referenzeinheiten, die weiter bevorzugt als Elektroden, noch weiter bevorzugt als Ringelektroden ausgebildet sind, aufweist, um so die entsprechende Anzahl von Potentialverläufen bei in einer Herzkavität eingeschobenen Arbeitskathetern zu erfassen.

17. System nach einem der vorstehenden Ansprüche,
wobei die Referenzeinheiten als Elektroden, bevorzugt als Ringelektroden ausgebildet sind.

18. System nach einem der vorstehenden Ansprüche,
wobei die bevorzugt als Elektroden, weiter bevorzugt als Ringelektroden ausgebildeten Referenzeinheiten gleichzeitig von der Verarbeitungseinheit (16) ansteuerbar sind.

19. System nach einem der vorstehenden Ansprüche,
wobei der Arbeitskatheter (10) mit einer Anzahl von mindestens zwei bevorzugt als Ringelektroden ausgebildeten Elektroden versehen ist, die örtlich verschieden von den Referenzeinheiten (4) auf dem Arbeitskatheter angebracht sind, wobei die Referenzeinheiten (4) zu den Elektroden eine zuvor festgelegte, bestimmte räumliche Lage aufweisen, die von der Verarbeitungseinheiten (16) bei der Ermittlung der Lage des Arbeitskatheters (10) in dem von dem Referenzkatheter (2) aufgespannten Koordinatensystem berücksichtigbar ist.

20. System nach einem der vorherigen Ansprüche, mit
Stellgliedem (24,26) am proximalen Ende des Arbeitskatheters (10), welche dazu ausgestaltet sind, eine Rotation des Arbeitskatheters (10) und/oder eine Biegung des distalen Ende (12) des Arbeitskatheters (10) zu bewirken.

21. System nach einem der vorherigen Ansprüche, mit
einer ersten Signalleitung (34), welche sich von der distalen Spitze (30) bis zum proximalen Ende des Arbeitskatheters (10) erstreckt und die mit den Arbeitskatheter-Referenzeinheiten (4a-c) verbunden ist,
einer zweiten Signalleitung (35), welche sich von der distalen Spitze bis zum proximalen Ende des Referenzkatheters (2) erstreckt und die mit den Referenzkatheter-Referenzeinheiten (14a,b) verbunden ist,
wobei die Verarbeitungseinheit (16) über die erste Signalleitung (34) mit den Arbeitskatheter-Referenzeinheiten (4) und über die zweite Signalleitung (35) mit den Referenzkatheter-Referenzeinheiten (14) verbunden ist,
wobei die Verarbeitungseinheit (16) mit den Stellgliedem (24, 26) verbunden und dazu ausgestaltet ist, die Stellglieder (24, 26) entsprechend der Signale von den Referenzkatheter-Referenzeinheiten (14a,b) anzusteuern, um eine Rotation bzw. eine Biegung des Arbeitskatheters (10) zu bewirken.

22. Arbeitskatheter mit einer distalen Spitze (30) und einem proximalen Ende zur Verwendung in einem System nach Anspruch 1,
**gekennzeichnet durch** Referenzeinheiten (4a-c), die dazu ausgestaltet sind, für die Position des Arbeitskatheters (10) charakteristische Signale zu senden, und
eine Signalleitung (34), welche sich von der distalen Spitze (30) bis zum proximalen Ende des Arbeitskatheters erstreckt und die mit den Referenzeinheiten (4a-c) verbunden ist.

23. Referenzkatheter mit einer distalen Spitze und einem proximalen Ende zur Verwendung in einem System nach Anspruch 1,
**gekennzeichnet durch** Referenzeinheiten (14a, b), die dazu ausgestaltet sind, Positionssignale zu empfangen, und
eine Signalleitung (35), welche sich von der distalen Spitze bis zum proximalen Ende des Referenzkatheters erstreckt und die mit den Referenzeinheiten (14a,b) verbunden ist.

## Claims

1. System for determining the intracorporal position of a working catheter (10), comprising a working catheter (10) for carrying out desired working operations, and an intracorporal reference catheter (2) for producing a coordinate system, **characterised in that** the working catheter (10) comprises a plurality of working catheter reference units (4a-c) for sending signals, which are characteristic of the position of the working catheter (10), and the reference catheter (3) comprises a plurality of reference catheter reference units (14) for receiving the signals sent by the working catheter reference units (4), the system also comprising a processing unit (16) for calculating the position and the intracorporal orientation of the working catheter (10) on the basis of signals received from the reference catheter reference units (14a, b).

2. System according to claim 1, wherein the working catheter (10) is a mapping catheter for producing a three-dimensional image of the heart cavity surrounding the mapping catheter.

3. System according to any one of the preceding claims, wherein the working catheter (10) is an ablation catheter for producing a preferably linear lesion of the endocardium surrounding the ablation catheter.

4. System according to any one of the preceding claims, wherein the working catheter (10) is a catheter which can be fixedly implanted in a body and which carries electrodes of a cardiac pacemaker or a defibrillator.

5. System according to any one of the preceding claims, wherein the working catheter reference units (4a-c) are asymmetrically arranged, preferably forming the corners of a triangle, on the working catheter (10), such that the orientation of the working catheter (10) can be detected in the coordinate system of the reference catheter (2).

6. System according to any one of the preceding claims, wherein the reference units (4, 14) are ultrasonic crystals or coils mounted on or in the catheter.

7. System according to any one of the preceding claims, wherein at least one reference unit (4a) is arranged at the catheter tip (30), while at least one further reference unit (4b, c) is arranged in the remaining distal region (12) of the catheter (10), preferably an entire series of reference units, more preferably 12 to 24 reference units, being arranged in the distal region (12).

8. System according to any one of the preceding claims, wherein the distal region (12) of the working catheter (10) has a predetermined, specific shape, preferably that of an arc of a circle, over which distal region at least three reference units (4) are distributed such that the specific, predetermined shape of the distal region (12) can be incorporated by the processing unit (16) when ascertaining the position of the working catheter (10) by calculating the position of the working catheter (10).

9. System according to any one of the preceding claims, wherein either the reference catheter (2) is also a working catheter (10) or the working catheter (10) is also a reference catheter (2), in that reference units (4, 14) for transmitting waves and reference units (4) for receiving waves, preferably electromagnetic and/or ultrasonic waves, are respectively provided on each catheter, and/or reference units (4, 14), which may simultaneously transmit and receive waves, preferably electromagnetic waves, are respectively provided.

10. System according to any one of the preceding claims, wherein the processing unit (16) carries out a topological and/or electrical measurement of the endocardium, in which the respective working catheter (10) is disposed, by means of the reference units (4).

11. System according to any one of the preceding claims, wherein the reference catheter reference units (14a, b) emit electromagnetic radiation and/or ultrasonic waves to ascertain the position of the working catheter (10) in the coordinate system spanned by the reference catheter (2), the reference catheter reference units (14a, b) building up at least one electromagnetic field.

12. System according to any one of the preceding claims, wherein the reference catheter (2) is preferably placed in the coronary sinus for use of the system in the heart.

13. System according to any one of the preceding claims, wherein the processing unit (16) calculates, on the basis of the data from the at least three working catheter reference units (4a-c), a three-dimensional spline that represents the position of the working catheter (10) in the coordinate system spanned by the reference catheter.

14. System according to any one of the preceding claims, wherein the processing unit (16) is integrated in the respective catheters.

15. System according to any one of the preceding claims, wherein at least one of the reference units is configured as a sensor for detecting the presence and/or the strength of the wall contact of the working catheter (10) with the endocardium surrounding the catheter.

16. System according to any one of the preceding claims, wherein the system comprises two, preferably five, working catheters (10), each catheter comprising at least three, preferably 12 to 24, reference units which are more preferably configured as electrodes, even more preferably as annular electrodes, for detecting the corresponding number of potential curves in the case of working catheters inserted into a cavity in a heart.

17. System according to any one of the preceding claims, wherein the reference units are configured as electrodes, preferably as annular electrodes.

18. System according to any one of the preceding claims, wherein the reference units, which are preferably configured as electrodes, more preferably as annular electrodes, may simultaneously be activated by the processing unit (16).

19. System according to any one of the preceding claims, wherein the working catheter (10) is provided with a number of at least two electrodes, which are preferably configured as annular electrodes and which are mounted on the working catheter at different locations from the reference units (4), the reference units being, relative to the electrodes, in a predetermined specific spatial position which can be taken into account by the processing units (16) when ascertaining the position of the working catheter (10) in the coordinate system spanned by the reference catheter (2).

20. System according to any one of the preceding claims, comprising control elements (24, 26) at the proximal end of the working catheter (10) for causing rotation of the working catheter and/or blending of the distal end (12) of the working catheter (10).

21. System according to any one of the preceding claims, comprising a first signal line (34) which extends from the distal tip (30) to the proximal end of the working catheter (10) and which is connected to the working catheter reference units (4a-c), a second signal line (35) which extends from the distal tip to the proximal end of the reference catheter (2) and which is connected to the reference catheter reference units (14a, b), wherein the processing unit (16) is connected via the first signal line (34) to the working catheter reference units (14) and via the second signal line (35) to the reference catheter reference units (14), the processing unit (16) being connected to the control elements (24, 26) and being configured for activating the control elements (24, 26) in accordance with the signals from the reference catheter reference units (14a, b) in order to cause rotation or bending of the working catheter (10).

22. Working catheter comprising a distal tip (30) and a proximal end for use in a system according to claim 1, **characterised by** reference units (14a, b) for sending signals which are characteristic of the position of the working catheter (10), and a signal line (34) which extends from the distal tip to the proximal end of the working catheter and which is connected to the reference units (14a, b).

23. Reference catheter comprising a distal tip and a proximal end for use in a system according to claim 1, **characterised by** reference units (14a, b) for receiving position signals, and a signal line (35) which extends from the distal tip to the proximal end of the reference catheter and which is connected to the reference units (14a, b).

## Revendications

1. Système pour déterminer la position intracorporelle d'un cathéter (10), équipé
d'un cathéter (10), agencé pour réaliser les processus souhaités, et
un cathéter de référence intracorporel (2), agencé pour produire un système de coordonnées,
**caractérisé en ce que** le cathéter (10) présente plusieurs unités de référence de cathéter (4a-c), réalisées pour émettre des signaux caractéristiques pour la position du cathéter (10), et
le cathéter de référence (3) présente plusieurs unités de référence de cathéter de référence (14), réalisées pour recevoir les signaux émis par les unités de référence (4) du cathéter,
le système présente en outre une unité de traitement (16) réalisée pour calculer la position et l'orientation intracorporelle du cathéter (10) conformément aux signaux reçus des unités de référence de cathéter de référence (14a,b).

2. Système selon la revendication 1, dans lequel le cathéter (10) est un cathéter de cartographie pour la réalisation d'une reproduction tridimensionnelle de la cavité cardiaque entourant le cathéter de cartographie.

3. Système selon l'une des revendications précédentes, dans lequel le cathéter (10) est un cathéter d'ablation pour générer une lésion de préférence linéaire de l'endocarde entourant le cathéter d'ablation.

4. Système selon l'une des revendications précédentes, dans lequel, concernant le cathéter (10), il s'agit d'un cathéter implantable de façon fixe dans un corps portant les électrodes d'un stimulateur cardiaque ou d'un défibrillateur.

5. Système selon l'une des revendications précédentes, dans lequel les unités de référence de cathéter (4a-c) sont disposées sur le cathéter (10) non symétriques, de préférence formant les coins d'un triangle, de sorte que l'orientation du cathéter (10) peut être détectée dans le système de coordonnées du cathéter de référence (2).

6. Système selon l'une des revendications précédentes, dans lequel, en ce qui concerne les unités de référence (4, 14), il s'agit de cristaux à ultrasons ou de bobines fixés sur ou dans le cathéter.

7. Système selon l'une des revendications précédentes, dans lequel au moins une unité de référence (4a) est disposée à la pointe du cathéter (30), tandis qu'au moins une autre unité de référence (4b,c) est disposée dans l'autre zone distale (12) du cathéter (10), dans lequel, dans la zone distale (12), de préférence toute une série d'unités de référence est disposée, en outre de préférence 12 à 24 unités de référence.

8. Système selon l'une des revendications précédentes, dans lequel la zone distale (12) du cathéter (10) présente une forme déterminée préalablement définie, de préférence celle d'un arc de cercle, dans la zone distale duquel au moins trois unités de référence (4) sont réparties, de sorte que la forme déterminée préalablement définie de la zone distale (12) peut être inclue par l'unité de traitement (16) lors de la détection de la position du cathéter (10) lors du calcul de la position du cathéter (10).

9. Système selon l'une des revendications précédentes, dans lequel, en ce qui concerne le cathéter de référence (2), il s'agit également d'un cathéter (10), ou, en ce qui concerne le cathéter (10), il s'agit également d'un cathéter de référence (2), tandis que, sur chaque cathéter, respectivement des unités de référence (4, 14) pour l'émission et des unités de référence (4) pour la réception d'ondes, de préférence des ondes électromagnétiques et/ou ultrasoniques, sont prévues et/ou respectivement des unités de référence (4, 14) sont prévues, qui peuvent simultanément émettre et recevoir des ondes, de préférence des ondes électromagnétiques.

10. Système selon l'une des revendications précédentes, dans lequel l'unité de traitement (16) est agencée, de manière à exécuter à l'aide des unités de référence (4) un mesurage topologique et/ou électrique de l'endocarde, dans lequel se trouve le cathéter respectif (10).

11. Système selon l'une des revendications précédentes, dans lequel les unités de référence de cathéter de référence (14a,b) sont réalisées, pour rayonner le rayonnement électromagnétique et/ou les ondes ultrasoniques, pour détecter la position du cathéter (10) dans le système de coordonnées fixé par le cathéter de référence (2), dans lequel les unités de référence de cathéter de référence (14a,b) constituent au moins un champ électromagnétique.

12. Système selon l'une des revendications précédentes, dans lequel le cathéter de référence (2) est placé de préférence dans le sinus coronaire lors d'une utilisation du système dans le coeur.

13. Système selon l'une des revendications précédentes, dans lequel l'unité de traitement (16) est réalisée, pour calculer à partir des données provenant d'au moins trois unités de référence de cathéter (4a-c) un spline tridimensionnel, qui représente la position du cathéter (10) dans le système de coordonnées fixé par le cathéter de référence.

14. Système selon l'une des revendications précédentes, dans lequel l'unité de traitement (16) est intégrée dans le cathéter respectif.

15. Système selon l'une des revendications précédentes, dans lequel au moins l'une des unités de référence est réalisée en tant que capteur pour la détection de l'existence et/ou de la solidité du contact avec la paroi du cathéter (10) avec l'endocarde entourant le cathéter.

16. Système selon l'une des revendications précédentes, dans lequel le système présente au moins deux, de préférence cinq, cathéters (10), dans lequel chaque cathéter présente au moins trois, de préférence 12 à 24 unités de référence, qui sont réalisées en outre de préférence sous la forme d'électrodes, de préférence sous la forme d'électrodes annulaires, afin de détecter la pluralité correspondante de déplacements de potentiel dans des cathéters introduits dans une cavité cardiaque.

17. Système selon l'une des revendications précédentes, dans lequel les unités de référence sont réalisées sous la forme d'électrodes, de préférence sous la forme d'électrodes annulaires.

18. Système selon l'une des revendications précédentes, dans lequel les unités de référence réalisées de préférence sous la forme d'électrodes, de façon plus préférable sous la forme d'électrodes annulaires, peuvent être commandées simultanément par l'unité de traitement (16) .

19. Système selon l'une des revendications précédentes, dans lequel le cathéter (10) est équipé d'une pluralité d'au moins deux électrodes réalisées de préférence sous la forme d'électrodes annulaires, qui sont montées sur le cathéter localement différentes des unités de référence (4), dans lequel les unités de référence (4) présentent par rapport aux électrodes une position spatiale déterminée préalablement définie, qui peut être prise en considération par les unités de traitement (16) lors de la détection de la position du cathéter (10) dans le système de coordonnées fixé par le cathéter de référence (2).

20. Système selon l'une des revendications précédentes, comportant des actionneurs (24, 26) à l'extrémité proximale du cathéter (10), qui sont réalisés de manière à effectuer une rotation du cathéter (10) et/ou une courbure de l'extrémité distale (12) du cathéter (10).

21. Système selon l'une des revendications précédentes, comportant
une première ligne signal (34), qui s'étend de la pointe distale (30) jusqu'à l'extrémité proximale du cathéter (10) et qui est reliée aux unités de référence de cathéter (4a-c),
une seconde ligne signal (35), qui s'étend de la pointe distale jusqu'à l'extrémité proximale du cathéter de référence (2) et qui est reliée aux unités de référence de cathéter de référence (14a,b),
dans lequel l'unité de traitement (16) est reliée aux unités de référence de cathéter (4) par l'intermédiaire de la première ligne signal (34) et aux unités de référence de cathéter de référence (14) par l'intermédiaire de la seconde ligne signal (35),
dans lequel l'unité de traitement (16) est reliée aux actionneurs (24, 26) et est réalisée pour commander les actionneurs (24, 26) conformément aux signaux provenant des unités de référence de cathéter de référence (14a,b), pour effectuer une rotation ou une courbure du cathéter (10).

22. Cathéter muni d'une pointe distale (30) et d'une extrémité proximale pour une utilisation dans un système selon la revendication 1,
**caractérisé par** des unités de référence (4a-c), qui sont réalisées pour émettre des signaux caractéristiques pour la position du cathéter (10), et
une ligne signal (34), qui s'étend de la pointe distale (30) jusqu'à l'extrémité proximale du cathéter et qui est reliée aux unités de référence (4a-c).

23. Cathéter de référence muni d'une pointe distale et d'une extrémité proximale pour une utilisation dans un système selon la revendication 1,
**caractérisé par** des unités de référence (14a,b) réalisées pour recevoir des signaux de position, et
une ligne signal (35), qui s'étend de la pointe distale jusqu'à l'extrémité proximale du cathéter de référence et qui est reliée aux unités de référence (14a,b).
